(19) Europäisches Patentamt
European Patent Office
Office européen des brevets

(11) Publication number: **0 268 269 B1**

(12) **EUROPEAN PATENT SPECIFICATION**

(45) Date of publication of patent specification: **16.06.93**

(51) Int. Cl.[5]: **C07C 25/22**, C07C 17/34, C07C 17/12

(21) Application number: **87116972.8**

(22) Date of filing: **17.11.87**

(54) **A method for preparation of polybromoacenaphthene and/or its condensate.**

(30) Priority: **18.11.86 JP 272959/86**

(43) Date of publication of application:
**25.05.88 Bulletin 88/21**

(45) Publication of the grant of the patent:
**16.06.93 Bulletin 93/24**

(84) Designated Contracting States:
**DE FR GB IT**

(56) References cited:
**FR-A- 2 533 916**

(73) Proprietor: **Tosoh Corporation**
**4560, Kaisei-cho**
**Shinnanyo-shi, Yamaguchi-ken(JP)**

(72) Inventor: **Okisaki, Fumio**
**2575, Oaza Tonda**
**Shinnanyo-shi Yamaguchi-ken(JP)**
Inventor: **Kubo, Masashige**
**6772, Oaza Tokuyama**
**Tokuyama-shi Yamaguchi-ken(JP)**
Inventor: **Sakka, Hideo**
**2-5-27, Hirano**
**Shinnanyo-shi Yamaguchi-ken(JP)**

(74) Representative: **Vossius & Partner**
**Siebertstrasse 4 P.O. Box 86 07 67**
**W-8000 München 86 (DE)**

**EP 0 268 269 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for the preparation of polybromoacenaphthene and/or its condensate (hereinafter abbreviated Con-BAN), which is a useful intermediate for the preparation of polybromoacenaphthylene and/or its condensate (hereinafter abbreviated Con-BACN), furthermore, it relates to a method for bromination of an acenaphthene derivative and/or its condensate (hereinafter abbreviated Con-AN). The structures of Con-AN, Con-BAN, and Con-BACN are shown in formulae (1), (2) and (3), respectively.

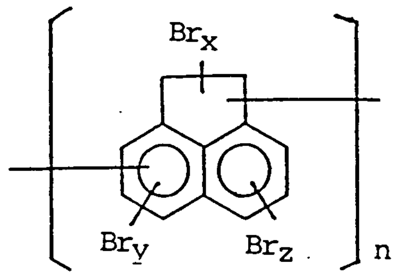

(1)

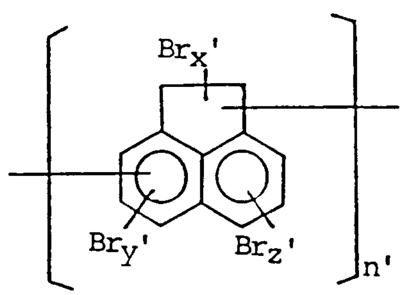

(2)

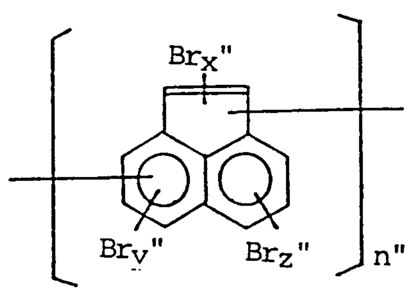

(3)

wherein in the formula (1) n is not less than 1, x, y and z are positive values respectively, and $x + y + z = 0 - 1$; in the formula (2) $n'$ is not less than 1, $x'$, $y'$ and $z'$ are positive values of 1 - 3 respectively, and $y' + z' = 2 - 6$; and in the formula (3) $n''$ is not less than 1, $x'' = 0 - 2$, both $y''$ and $z''$ are positive values of 1 - 3 respectively, and $y'' + z'' = 2 - 6$.

Con-BACN possesses an excellent property in that it will bring about flame retardancy and radiation resistance to high molecular substances when mixed in various resins and rubbers. The compound has been furthermore found to give rise to graft polymerization with high molecular substances by producing free radicals due to the double bond in the molecule. Since the compound is a condensate and hence superior in compatibility to high molecular substances, flame retardancy and radiation resistance can be stably maintained over a long period (Japanese Laid-Open Patent Application No. Sho 56-122862). On the other hand, radiation resistance as well as flame retardancy is a requirement for various resin constituents utilized in the coating material for an electric wire cable, which is utilized in a nuclear reactor, a breeder reactor, or ionic radiation generator. Con-BACN, therefore, has been expected to be utilized as a material to those matters.

Con-BACN from the present invention is a compound containing at least one or more bromine groups in an aromatic ring, and is formally a mixture of a monomer and a condensed product having such a structure as illustrated by formula (3). It can preferably be prepared as follows: polybromoacenaphthene is condensed by Friedel-Crafts alkylation to produce a mixture containing polymers having a degree of condensation not less than 2, followed by dehydrobromination. The mode of the bonding between the repeating units of the condensate is a single bond between the vinyl carbon and aryl carbon. The location of the bond can be exemplified (when representing the carbon skeleton only in the compound with the degree of condensation of 2) to be placed:
between 1 (or 2) and 3′

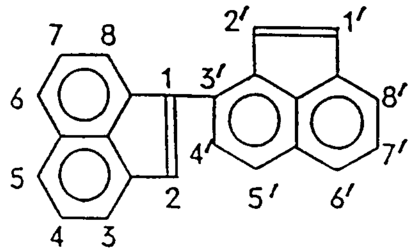

or between 1 (or 2) and 5′

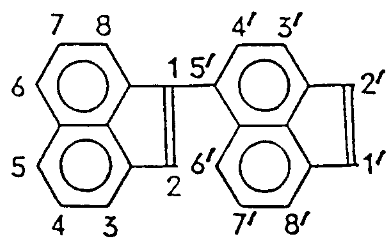

Other locations are considered to be 1 (or 2) - 4′, 1 (or 2) - 6′, 1 (or 2) - 7′, 1 (or 2) - 8′. In case the degree of condensation is 3 or more, the number of repeating units is increased by any of such bondings. Con-BACN from the present invention is a compound with a degree of condensation of not more than 10. It exhibits an excellent compatibility with high molecular substances.

Con-BACN can be prepared by dehydrobromination of Con-BAN as expressed in the formula (2) (Japanese Laid-open Patent Applications No. Sho 60-139630 and No. Sho 60-178832).

FR-A-2 533 916 relates to a process for the production of bromoacenaphthylene condensation products which process comprises the bromination and condensation of acenaphthene by bromine in a halogenated hydrocarbon solvent in the presence of a LEWIS catalyst.

A method for the preparation of Con-BAN, where a Lewis acid catalyst is added to a halogenated hydrocarbon solution of Con-AN as illustrated in formula (1) and where bromination is carried out by dropwise addition of bromine, has already been known from Japanese Laid-Open Patent Application No. Sho 59-221313. This method is industrially useful in that the reaction solution from the processes of producing Con-AN can be further employed without any separation procedures.

Incidentally, Con-AN from the present invention can be prepared by the reaction illustrated in the following reaction scheme (4), according to Japanese Laid-Open Patent Application No. Sho 59-221313. Here the side chain of acenaphthene is brominated in a halogenated hydrocarbon solution either by ultraviolet irradiation or in the presence of a radical initiating agent, and Friedel-Crafts alkylation is subsequently conducted by adding a Lewis acid catalyst to the resultant reaction solution.

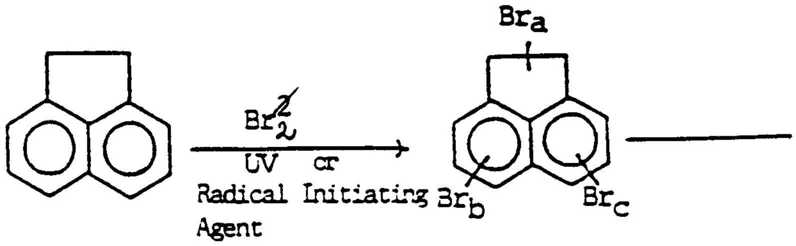


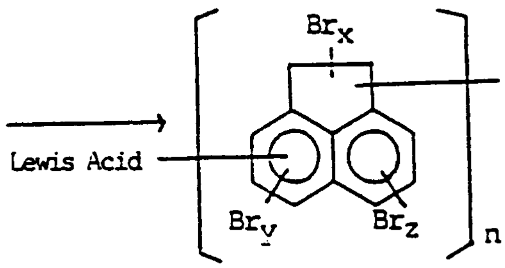


(4)

In the above general formulae

x, y, z and n are the same as in the formula (1), a is a positive value of 0 - 2, b and c are positive values of 0 - 1 respectively, and b + c = 0 - 1.

In the conventional method, however, the condensation reaction proceeds further as bromination does, hence it is difficult to control the degrees of both condensation and bromination. That is, there is a problem that under the condition which increases the degree of bromination, the condensation reaction advances excessively producing polybromides and/or substances having a high degree of condensation which sequentially deposit as insoluble substances from the reaction system

due to their low solubility.

The quantity of the insoluble substance thus produced, though depending on reactive conditions, reaches 20 weight percent against the product Con-BACN in the case of production of Con-BACN suitable for ethylene-propylene-dien rubber, hence the yield is about 75 percent, which is far from being satisfactory. Furthermore, the insoluble substance has such a high melting point that homogeneous dispersion into high molecule substances is decreased so that the aforesaid radiation resistance can not be obtained. Therefore, it was desired to find a method where the rate of by-production of insoluble substances is inhibited. This can be achieved by preparing Con-BAN through the bromination of Con-AN according to the invention.

The present inventors investigated methods of bromination of Con-AN, in which the by-production of aforesaid insoluble substance can be inhibited, and completed the present invention, a method for preparation of Con-BAN, in which Con-AN is reacted with bromine in the presence of Lewis acid catalyst and the solution of Con-AN and bromine are supplied simultaneously at a given rate into a reactor in which halogenated hydrocarbon and iron catalysts are previously introduced, and reacted to prepare Con-BAN.

Therefore, the present invention relates to a method for the preparation of polybromoacenaphthene and/or its condensate (Con-BAN) of formula (2), characterized in that a solution of an acenaphthene derivative and/or its condensate (Con-AN) of formula (1) and bromine are supplied simultaneously at a given rate into a reactor in a feed pace ratio based on moles of bromine to the acenaphthene unit structure of the acenaphthene derivative and/or condensate of 5 to 15 and a molar ratio of introduced bromine to the acenaphthene unit structure of the acenaphthene derivative and/or condensate of 3 to 6, at a temperature of 0° to 50°C, to which a halogenated hydrocarbon and iron catalysts are previously supplied, and the reaction mixture is allowed to react with the bromine employed to form the polybromoacenaphthene and/or its condensate illustrated in formula (2)

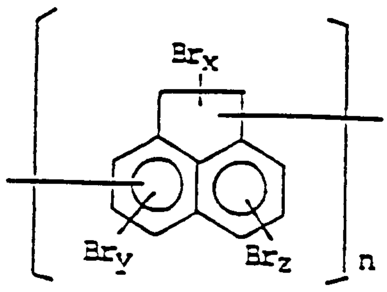

(1)

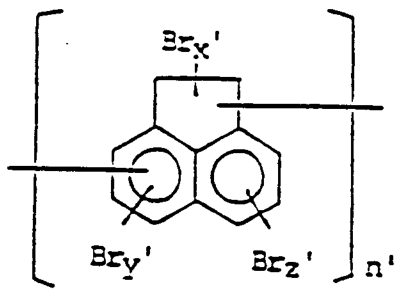

(2)

wherein in the formula (1) n is not less than 1, and x, y and z are positive values respectively, and x + y + z = 0 - 1; in the formula (2) n' is not less than 1, x', y' and z' are positive values of 1 - 3 respectively, and y' + z' = 2 - 6.

According to the method of the present invention, it is possible to obtain the Con-BAN having the same quality as in the conventional method in higher yields, and at the same time formation of the aforesaid insoluble substances can be suppressed.

The reason why such action is brought about by the present invention is not completely understood, but the following assumption is discussed: In this method, the molar ratio of bromine to acenaphthene unit structure can be controlled over the whole period of mixing these two raw material solutions by properly determining the feed pace ratio in molar basis (hereinafter abbreviated as FPR) of bromine to the acenaphthene unit structure of Con-AN, and the molar ratio of introduced bromine to the acenaphthene unit structure (hereinafter abbreviated as B/A ratio).

Under the conditions of such as FPR and B/A ratios where an excess of bromine is present per acenaphthene unit structure, bromination, especially the rate of bromination in the aromatic ring, is relatively increased in the initial stage of the mixing and reaction period. Therefore, since the sites which are active to electrophilic attack in the aromatic ring of the acenaphthene unit structure are filled with bromine atoms, the condensation reaction is inhibited and hence the production of insoluble substances or polycondensate is considered to be restricted.

Therefore, both the FPR and B/A ratio are essential to be more than a given value, preferably the FPR value ranging from 5 to 15 and the B/A ratio from 3 to 6. If the FPR ratio is less than 5 or the B/A ratio is less than 3, the promotion of bromination as mentioned above proceeds insufficiently while the condensation proceeds. In this case the quantity of insoluble substances increases. Furthermore, if the FPR ratio is more than 15 or the B/A ratio is more than 6, the bromination advances at high degree and polybromides with lower solubility are deposited, thus increasing the formation of insoluble substances. In case the B/A ratio is more than 6, a large excess of bromine remains in the reaction system after completion of the reaction, resulting in the requirement of complicated procedures for the elimination of excessive bromine.

Bromination of the present invention is an exothermic reaction, hence it is desired to facilitate elimination of the heat generated by the reaction. In order to prevent the thermal promotion of the condensation reaction, the feed rate of Con-AN is desirable to be 1.0 or less mol-acenaphthene unit structure/total acenaphthene unit structure/hr, and the feed rate of bromine to be 6.0 or less Mol-Br/total acenaphthene unit structure/hr.

The concentration of the Con-AN solution supplied to the reactor is preferably 1 to 50 weight percent. Concentrations of more than 50 percent promote the condensation reaction or a reaction of Con-AN with itself , and a concentration of less than 1 percent is not economical because the quantity of solvent used increases.

The concentration of the bromine solution supplied to a reactor is preferably not less than 50 weight percent. A concentration of less than 50 percent is not economical because much solvent is required. There is no problem when bromine is employed in an undiluted form, which is particularly desirable from the economical point of view, because the quantity of solvent used decreases.

Any solvent for Con-AN solution and bromine solution supplied in a reactor is permitted, provided that it is inactive to the claimed reaction and suitable to dissolve the raw material and products adequately. Halogenated hydrocarbons are preferred because of their inert character towards the reaction, their solubility and availability. The reaction proceeds smoothly even though Con-AN and bromine are dissolved in different solvents, but from the point or view of a desired recovery of the solvent, the same solvent should be preferably employed. Preferred solvents are: carbon tetrachloride, chloroform/dichloromethane, carbon tetrabromide, bromoform, dibromomethane, 1,2-dichloroethane, 1,1,2-trichloroethane.

The quantity of halogenated hydrocarbon previously introduced in the reactor is preferably 0.1 to 1.0 calculated on a volume ratio of the total volume of solvent supplied to the raw material solutions. If less than 0.1 are introduced, a stable stirring in the reaction vessel cannot be achieved during the initial stage of the supply of raw material solutions, and there is an extreme temperature rise due to liberated heat of the reaction, so that it becomes difficult to control the reaction. On the other hand, a volume ratio of more than 1.0 is undesirable from the economical point of view, because then the quantity of solvent employed increases.

As for iron catalysts, those which are usually used as Lewis acid catalysts are preferred, but substances or mixtures consisting of a plurality of substances selected from the following group are particularly desirable: iron powder, ferrous chloride, ferrous bromide, ferrous iodide, ferrous nitrate, ferrous sulfate, iron sulfide, ferrous oxide, ferric chloride, ferric bromide, ferric iodide, ferrocene, iron phthalocyanine and iron ehtylenediaminetetraacetate; most preferably ferric chloride.

The quantity of iron catalysts previously introduced into the reactor is preferably 0.1 to 5 mol percent based on the acenaphthene unit structure. In case the quantity is less than 0.1 mol percent, the bromination does not proceed adequately. On the other hand, in case of more than 5 mol percent iron catalyst, the bromination proceeds at a high degree, polybromides with low solubility are deposited, and the formation of resultant insoluble substances increases. This quantity has been furthermore found to lower the heat stability of the final product Con-BACN, the reason remaining unclear, so that the merchandise value of such products would be extremely impaired.

The temperature in the reactor to which both the Con-AN solution and bromine are supplied is preferably 0 °C to 50 °C. With temperatures higher than 50 °C, the condensation reaction is promoted and bromine is evaporated to an extreme degree. Since this reaction is an exothermic reaction, it is not economical to control the temperature to lower than 0 °C. According to the combination of the B/A ratio and the FPR ratio, the time when the Con-AN solution is supplied and bromine is added does not coincide, hence either the Con-AN solution or bromine will inevitably be supplied at a later stage, the temperature being preferably 0 ° to 50 °C even at that time. Incidentally, there is no problem if the temperature of the reaction system will be raised after completing the supply of the two raw material solutions, in order to promote the conversion of bromine.

Detailed Description of the Preferred Embodiments

The following Examples and Comparative Examples will describe the present invention in detail, but the present invention is not limited thereto.

The production ratio of insoluble substances in Examples and Comparative Examples is the quantity of insoluble substance, which is obtained by the following formula (5).

$$\text{Ratio of Insoluble Substances} = \frac{\text{Quantity of Insoluble Substances}}{(\text{Con-BACN Yield}) + (\text{Quantity of Insoluble Substances})} \times 100 \qquad (5)$$

The average degree of condensation $\overline{DC}$ was calculated by substituting the peak area rate Ai (i is the degree of condensation) in components of each degree of condensation for the formula (6), after determining the distribution of the degree of condensation by gel permeation chromatography (GPC).

$$\overline{DC} = \sum_{i} i \times Ai \qquad (6)$$

GPC analysis was carried out under the following conditions.

Column : Tosoh Corporation TSK - Gel G1000H
Detector: Tosoh Corporation Differential Refractometer R1 - 8
Eluent: Tetrahydrofuran

The thermal decomposition rate is expressed in weight percent of HBr, which generates when Con-BACN is heated at 160 °C for 3 hours, against the weight of Con-BACN employed in the experiment.

Examples 1 to 7, and Comparative Examples 1, 2:

Con-AN solutions employed in Examples 1 to 7 and Comparative Examples 1 and 2 were prepared according to the following procedures: Acenaphthene 77.1 g (0.500 mol) and 1.64 g of $\alpha,\alpha'$-azobisisobutyronitril (10 mmol) were dissolved in 351 g of carbon tetrachloride in a 1-liter flask with four necks under dry nitrogen atmosphere, to which a solution, in which 52.0 g (o.375 mol) of bromine had been dissolved in 237 g (the concentration of bromine is 18 wt-%) of carbon tetrachloride, was dropped in 50 min with stirring and refluxing, and then refluxing was continued for more than 30 min. After cooling to room temperature, a solution of titanium tetrachloride 4.74 g (25 mmol) dissolved in carbon tetrachloride 42.7 g was added in 10 min to the reaction solution. The mixture was kept stirring at room temperature for 1 hr. The resulting reaction solution contained 81.1 g of Con-AN, $\overline{DC}$ 2.30 and its bromine content was 5.6%, concentration 11% by weight, acenaphthene unit structure 0.500 mol).

Bromination of Con-AN was carried out by using the reaction solution corresponding to 0.5 mol of acenaphthene thus obtained, according to the following methods: 200 g of carbon tetrachloride were introduced in a 1-liter flask with four necks and ferric chloride was added as a catalyst at quantities shown in Table 1, into which the Con-AN solution and bromine were supplied simultaneously while stirring vigorously at temperatures shown in Table 1. Con-AN was supplied in the condition of 0.14 mol-acenaphthene unit structure/hr. Bromine was added without being diluted in a solvent at feeding rates determined from the FPR and the B/A ratio as shown in Table 1. In Examples 1 to 7 and Comparative Examples 1 and 2, even after completing the supply of bromine, Con-AN was supplied sequentially. After completing the supply of Con-AN, stirring was continued at the same temperature for 0.5 hr, and then heat aging with refluxing was accomplished for periods as shown in Table 1. The reaction solution, after separating it from an insoluble substance by filtration, was washed with water and after unreacted bromine was reduced by a solution of sodium hydrogen sulfite (25 wt-%), was further washed to obtain a solution of Con-BAN in carbon tetrachloride. Incidentally, the insoluble substance was washed out with carbon tetrachloride, and after separation, dried out at 120 °C for 8 hr . The by-production rate of insolubles was calculated. Results are summarized in Table 1.

Subsequently, the Con-BAN solution was refluxed with a potassium hydride solution for 2 hr (where 56.1 g of potassium hydride (1.00 mol) were dissolved in 215 ml of methanol) and then washed with water and with 5% of sulfuric acid and finally with water, to obtain a solution of Con-BACN in carbon tetrachloride.

To the Con-BACN solution was added 0.29 g of polyoxyethylene (20) sorbitan monopalmitate (Wako Pure Chemical Industries, Ltd.) as a surface active agent. This solution was added dropwise to 1.5 liter of water, in which 0.14 g of the surface active agent had been dissolved, and heated 3 hr at 95 °C. By this treatment carbon tetrachloride in the Con-BAN solution was eliminated due to azeotropy with water. The precipitated Con-BACN powder was filtered off and dried under normal pressure at 120 °C for 8 hr. The yield, yield percent values, $\overline{DC}$, bromine content, and heat decomposition rate of the powder are summarized in Table 2.

Comparative Example 3:

Into the Con-AN solution (acenaphthene unit structure 0.500 mol) obtained by the same procedure as that in Examples 1 to 7, 384 g of bromine (2.40 mol) were added dropwise at 30 °C in 3.5 hr, and stirring was accomplished for 0.5 hr at the same tempature, whereupon the mixture was further heated for 6 hr under refluxing. The obtained reaction mixture was treated with the same method as in Examples 1 to 7. The results are shown in Tables 1 and 2.

Table 1

| | B/A Ratio | FPR | Catalyst (mol %) | Temperature of dropwise addition (°C) | Reflux Maturation (hr) | Insoluble Substance (wt-%) |
|---|---|---|---|---|---|---|
| Example 1 | 4.8 | 10 | 1 | 30 | 6 | 3.40 |
| Example 2 | 4.8 | 7.5 | 3 | 30 | 6 | 4.40 |
| Example 3 | 4.8 | 10 | 3 | 30 | 6 | 5.05 |
| Example 4 | 4.8 | 15 | 3 | 30 | 5 | 5.79 |
| Example 5 | 4.6 | 10 | 1 | 30 | 6 | 3.15 |
| Example 6 | 5.0 | 10 | 1 | 30 | 6 | 5.53 |
| Example 7 | 4.8 | 10 | 1 | 50 | 6 | 4.00 |
| Comparative Example 1 | 4.8 | 20 | 3 | 30 | 5 | 12.19 |
| Comparative Example 2 | 4.8 | 10 | 1 | 60 | 6 | 10.21 |
| Comparative Example 3 | 4.8 | 0 | 5 | 30 | 6 | 19.50 |

Table 2

| | Yield (g) | Yield (%) | $\overline{DC}$ | Bromine Content (wt-%) | Pyrolysis Rate (wt-%) |
|---|---|---|---|---|---|
| Example 1 | 180.59 | 89.5 | 2.43 | 63.2 | 0.03 |
| Example 2 | 176.43 | 85.0 | 2.47 | 64.0 | 0.04 |
| Example 3 | 182.13 | 85.1 | 2.44 | 64.2 | 0.06 |
| Example 4 | 187.48 | 85.3 | 2.30 | 64.5 | 0.09 |
| Example 5 | 171.82 | 89.6 | 2.40 | 61.3 | 0.04 |
| Example 6 | 179.70 | 88.3 | 2.43 | 63.5 | 0.04 |
| Example 7 | 179.32 | 88.3 | 2.48 | 63.4 | 0.03 |
| Comparative Example 1 | 187.72 | 79.1 | 2.30 | 67.0 | 0.18 |
| Comparative Example 2 | 177.78 | 79.8 | 2.54 | 63.3 | 0.04 |
| Comparative Example 3 | 153.20 | 75.9 | 2.43 | 63.2 | 0.08 |

It has been definitely shown from the results of the Examples and Comparative Examples that the method of the present invention enables the preparation of Con-BACN having the same quality as in the conventional method, while it simultaneously suppresses the formation of insoluble substances to an extreme extent in the stage of the preparation of Con-BAN from Con-AN, and hence the inventive method enhances the total yield of Con-BACN produced from acenaphthene.

## Claims

**1.** A method for the preparation of polybromoacenaphthene and/or its condensate (Con-BAN) of formula (2), characterized in that a solution of an acenaphthene derivative and/or its condensate (Con-AN) of formula (1) and bromine are supplied simultaneously at a given rate into a reactor in a feed pace ratio based on moles of bromine to the acenaphthene unit structure of the acenaphthene derivative and/or condensate of 5 to 15 and a molar ratio of introduced bromine to the acenaphthene unit structure of the acenaphthene derivative and/or condensate of 3 to 6, at a temperature of 0° to 50°C, to which a halogenated hydrocarbon and iron catalysts are previously supplied, and the reaction mixture is allowed to react with the bromine employed to form the polybromoacenaphthene and/or its condensate illustrated in formula (2)

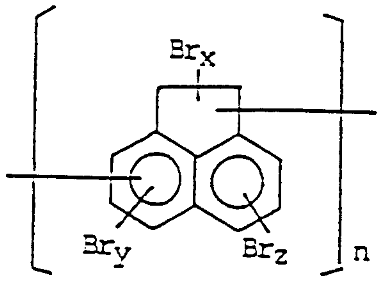

(1)

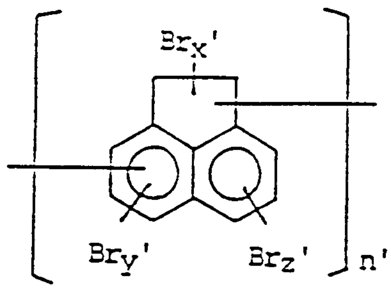

(2)

wherein in the formula (1) n is not less than 1, and x, y and z are positive values respectively, and x + y + z = 0 - 1; in the formula (2) n' is not less than 1, x', y' and z' are positive values of 1 - 3 respectively, and y' + z' = 2 - 6.

**2.** A method according to Claim 1, wherein the iron catalyst is iron powder, ferrous chloride, ferrous bromide, ferrous iodide, ferrous nitrate, ferrous sulfate, iron sulfide, ferrous oxide, ferric chloride, ferric bromide, ferric iodide, ferrocene, iron phthalocyanine or iron ethylenediaminetetraacetate.

**3.** A method according to Claim 1, wherein the iron catalyst is ferric chloride.

**4.** A method according to Claim 1, wherein the amount of the iron catalyst is 0.1 to 5 mol % with respect to the acenaphthene unit structure of the acenaphthene derivative and/or condensate.

**Patentansprüche**

**1.** Verfahren zur Herstellung von Polybromacenaphthen und/oder dessen Kondensat (Con-BAN) der Formel (2), **dadurch gekennzeichnet**, daß man eine Lösung eines Acenaphthenderivats und/oder dessen Kondensats (Con-AN) der Formel (1) und Brom gleichzeitig mit einer bestimmten Geschwindigkeit bei einer Temperatur von 0 bis 50 °C und einem Zudosierungsgeschwindigkeitsverhältnis, bezogen auf Mole Brom zu der Acenaphthenstruktureinheit des Acenaphthenderivats und/oder dessen Kondensats, von 5 bis 15 und einem Molverhältnis von eingeführtem Brom zu der Acenaphthenstruktureinheit des Acenaphthenderivats und/oder dessen Kondensats von 3 bis 6 in einen Reaktor einspeist, in den vorher ein halogenierter Kohlenwasserstoff und Eisenkatalysatoren gegeben wurden, und daß man das Reaktionsgemisch mit dem eingesetzten Brom zur Erzeugung des Polybromacenaphthens und/oder dessen Kondensats der Formel (2) reagieren läßt:

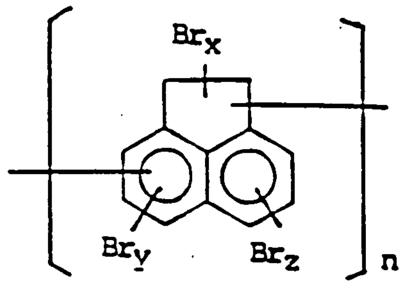

(1)

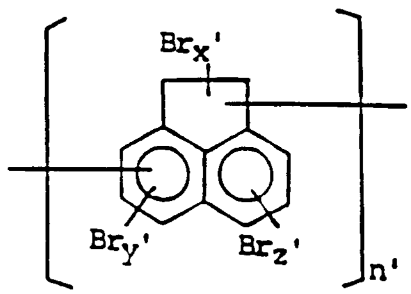

(2)

wobei in Formel (1) n nicht kleiner als 1 ist und x, y und z jeweils positive Werte darstellen, und x + y + z = 0 - 1; und wobei in der Formel (2) n' nicht kleiner als 1 ist, x', y' und z' jeweils positive Werte von 1 bis 3 darstellen und y' + z' = 2 - 6.

**2.** Verfahren gemäß Anspruch 1, wobei der Eisenkatalysator Eisenpulver, Eisen(II)-chlorid, Eisen(II)-bromid, Eisen(II)-jodid, Eisen(II)-nitrat, Eisen(II)-sulfat, Eisensulfid, Eisen(II)-oxid, Eisen(III)-chlorid, Eisen-(III)-bromid, Eisen(III)-jodid, Ferrocen, Eisenphthalocyanin oder Eisenethylendiamintetraacetat ist.

**3.** Verfahren gemäß Anspruch 1, wobei der Eisenkatalysator ein Eisen(III)-chlorid ist.

**4.** Verfahren gemäß Anspruch 1, wobei die Menge des Eisenkatalysators 0,1 bis 5 Mol-%, bezogen auf die Acenaphthenstruktureinheit des Acenaphthenderivats und/oder Kondensats beträgt.

**Revendications**

**1.** Procédé pour la préparation de polybromoacénaphtène et/ou de son produit de condensation (Con-BAN) de la formule (2), caractérisé en ce qu'une solution d'un dérivé d'acénaphtène et/ou de son produit de condensation (Con-AN) de la formule (1) et du brome sont fournis simultanément à un taux donné dans un réacteur dans un rapport de l'allure d'alimentation basé sur les moles de brome et la structure du motif d'acénaphtène du dérivé d'acénaphtène et/ou du produit de condensation égal à 5 - 15 et un rapport molaire du brome introduit et de la structure du motif d'acénaphtène du dérivé d'acénaphtène et/ou de son produit de condensation égal à 3 - 6, à une température de 0° à 50°C, auquel on a fourni au préalable un hydrocarbure halogéné et des catalyseurs en fer, et on a laissé le mélange réactionnel réagir avec le brome employé pour former le polybromoacénaphtène et/ou son produit de condensation représenté dans la formule (2):

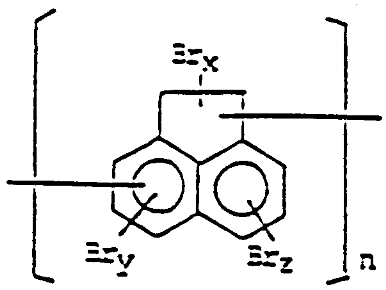

(1)

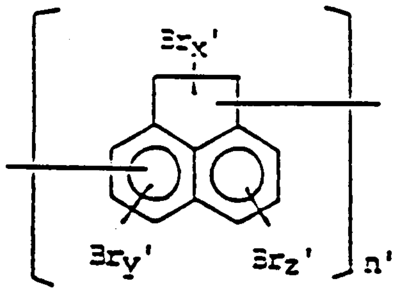

(2)

dans laquelle, en formule (1), n n'est pas inférieur à 1, et x, y et z sont des valeurs positives respectivement, et x + y + Z = 0 - 1; en formule (2), n' n'est pas inférieur à 1, x', y' et z' sont les valeurs positives 1 - 3, respectivement, et y' + z' = 2 - 6.

2. Procédé selon la revendication 1, dans lequel le catalyseur en fer est la poudre de fer, le chlorure ferreux, le bromure ferreux, l'iodure ferreux, le nitrate ferreux, le sulfate ferreux, le sulfure de fer, l'oxyde ferreux, le chlorure ferrique, le bromure ferrique, l'iodure ferrique, le ferrocène, la phtalocyanine de fer ou l'éthylènediaminetétraacétate de fer.

3. Procédé selon la revendication 1, dans lequel le catalyseur en fer est le chlorure ferrique.

4. Procédé selon la revendication 1, dans lequel la quantité du catalyseur en fer est de 0,1 à 5 moles % par rapport à la structure du motif d'acénaphtène du dérivé d'acénaphtène et/ou de son produit de condensation.